# EUROPEAN PATENT SPECIFICATION

(11) **EP 1 323 832 B1**
(45) Date of publication and mention of the grant of the patent: **05.07.2006**
(21) Application number: 01964834.4
(22) Date of filing: 24.08.2001
(51) Int. Cl.: C12Q 1/04, C12R 1/04, C12R 1/19, C12R 1/22, C12R 1/37, C12R 1/185, C12R 1/42

(54) **CULTURE MEDIUM AND METHOD FOR IDENTIFYING GRAM-NEGATIVE MICROORGANISMS**
KULTURMEDIUM UND VERFAHREN ZUR BESTIMMUNG GRAM-NEGATIVER MIKROORGANISMEN
MILIEU DE CULTURE ET METHODE DESTINES A L'IDENTIFICATION DE MICRO-ORGANISMES GRAM-NEGATIFS

(30) Priority: 07.09.2000 CU 1952000
(43) Date of publication of application: 02.07.2003
(73) Proprietor: CENTRO NACIONAL DE BIOPREPARADOS, Bejucal, Provincia La Habana 6048 (CU)
(72) Inventor: RODRIGUEZ MARTINEZ, Claudio, Ciudad de la Habana (CU); QUESADA MUNIZ, Vivian de Jesus, Ciudad de la Habana (CU); ZHURBENKO, Raisa, Ciudad de la Habana (CU)
(74) Representative: Prins, Adrianus Willem
(86) International application number: PCT/CU2001/000006
(87) International publication number: WO 2002/020829

(56) References cited:
- WO-A-96/30543
- ES-A- 2 079 319
- US-A- 5 194 374
- HENGSTLER K A ET AL: "Evaluation of BBL CHROMagar orientation medium for detection and presumptive identification of urinary tract pathogens." JOURNAL OF CLINICAL MICROBIOLOGY, vol. 35, num. 11, Noviembre 1997 (1997-11), páginas 2773-2777, XP002902211

## Description

### Technical Sector

The present invention is related with the field of the microbiological diagnosis, and more specifically with the identification or differentiation of Gram-negative bacteria.

### Prior Art

The diagnosis of Gram-negative bacteria has a crucial importance for the human's and animal's health and for the preservation of the environment, since many of them, such as *E. coli, Salmonella, Klebsiella, Pseudomonas,* causes serious illnesses in the man.

For more than 100 years, different culture media have been developed for the identification and count of these bacteria.

Starting from the decade of the 1990, new culture media were developed with the use of chromogenic and/or fluorogenic substrates to identify, in a better way, some of these pathogens.

Ferguson in 1994 (Patent No. US 5,358,854 of 1994), protected an invention consistent in a culture media and chromogenic reagents for the identification and differentiation of *E. coli* and coliforms. The essence of the invention consisted on using a substrate for the enzyme beta-galactosidase that produced an insoluble precipitate of a first color and a substrate beta-glucuronidase that caused the appearance of a second color. The medium only allows the coliforms differentiation in general, but do not allowed the differentiation between them.

Alain Rambach patented in 1993 (Patent No. US 5,194,374) a medium for the isolation of *Salmonella* based on the capacity of this microorganism to metabolize the polyoles. The medium does not allow the identification of a wide variety of genera and species of coliforms of a great diagnostic interest.

In that year, F. Denis and coworkers (Évaluation d'un nouveau milieu avec substrats chromogénes pour la recherche de salmonelles dans des coprocultures: un milieu SMID., Revue française des laboratoires, decembre 1994, No. 271, pp. 19-22) published the evaluation of a chromogenic medium for the isolation and the pre-identification of *Salmonella*. The medium consist of 2 chromogenic substrates, one that stain the colonies with a blue color based on the β-galactosidase activity, and another that stain the colonies of *Salmonella* with a red color, based on the degradation of the glucuronate that combines with a color indicator.

With the help of this medium, *Salmonella typhi, Salmonella paratyphi* B and

*Salmonella typhimurium, Proteus, Citrobacter, E. coli* and *Enterobacter* can be differentiated. The medium contains potent inhibitors for the microorganisms, such as crystal violet and the bile salts, and it requires the employment of a TRIS buffer, that makes more complex and inhibitory the medium, even for some organisms of interest. On the other hand, Polytone is used. It is a complex mixture of protein hydrolysates. Lastly, the medium doesn't allow the identification of organisms of great interest, such as *Pseudomonas, E. coli* O157:H7, among others.

Rambach in 1997 patented a culture medium and the method for the detection of the strains of Enterohemorrhagic *E. coli* (Patent application No. WO 97/39103). The invention consists on a selective medium for the differentiation of *E. coli*, especially of the serotypes O157 and/or O11 that utilize a chromogenic substrate for the enzyme *β-galactosidase*. With the purpose of increasing the differential capacity of the method, it ware used other chromogenic substrates for β-glucosidase which is hydrolyzed by a great number of coliform bacteria and for β-glucuronidase which is hydrolyzed by *E. coli* serogroups different from O157 and O11. This culture medium does not facilitate the differentiation of other Gram-negative bacteria and, Wallace and Jones reported that some strains of *E. coli* and *Citrobacter* could give false-positive results (Wallace and Jones, J. Appl. Bacteriol., 1996, 81: 663-668).

Rambach years later, in 1998, patented a new culture medium for the detection of *E. coli* (Patent No. US 5,846,761), based on the use of a chromogenic substrate derived from the indolyl-glucuronic acid and their salts. This specific medium does not allow the identification of strains of *E. coli* O57:H7 neither of other coliforms.

In 1995 Monget and collaborators (Patent No. US 4,277,561) patented a method for the bacteriological analysis and a medium for the detection of *Salmonella.* The method is based on the capacity of the *Salmonella* to ferment the glucuronic acid or its salts and not to produce the enzyme β-galactosidase. The medium also contained nutrients, a fluorogenic or chromogenic compound for the enzyme β-galactosidase, glucuronic acid or one of its salts and a pH indicator.

This medium also contains inhibitors of the genera and species different from *Salmonella*, such as brilliant green and sodium deoxycholate, which can inhibit other

Gram-negative organisms. The culture medium requires the addition of the sodium glucuronate as a supplement after sterilization. According to Denis and collaborators, the specificity of this medium is smaller than 94 % (93,3 %), (Denis, et al, Revue francaise des laboratoires, décembre 1994, No. 271).

Tuompo and collaborators patented, on the other hand, a method and medium for cultivation and identification of *Salmonella* based on the use of melibiose, mannitol and sorbitol and of substrates for beta-glucuronidase (Patent No. US 5,786,167. Its brown, blue or green color, identified the rest of the coliforms in dependence of the chromogenic substrate used. With this method, the authors were not able to appropriately differentiate to each other the more relevant coliforms organisms.

Miller and collaborators in 1999 (Patent No. US 5,871,944 of 1999), patented a medium to differentiate *Salmonella* using lactose and cellobiose to suppress the appearance of black precipitates in the medium, and thus allows the detection of the presence or absence of *Salmonella*. The medium requires the employment of a TRIS buffer, peptones and meat extract, X-gal and other ingredients.

This medium is only appropriate for some Gram-negative bacteria, such as *Salmonella* and *Shigella*, these last can only be differentiated as such, and do not between them.

Butchner patented in 1996 a medium and a method for the isolation and the presumptive identification of several bacteria, in particular, those that cause urinary sepsis or urinary infections. (Patent No. US 5,541,082). The method bases its principle, in distinguishing them by the colonial morphology and the color.

The medium contains a proteinaceous opaque material, two or more chromogenic substrates, arylsulphatases, galactosidases, glucuronidases, tryptophan-oxidases and tiramino-oxidases, also, it is not specific for the Gram-negative bacteria that are indicative of the quality of the foods and waters.

On the other hand, with their use, organisms of great clinical and sanitary importance cannot be properly identified, among them, *E. coli* O157:H7, *Serratia, Citrobacter, Aeromonas*, and others. Also, are needed additional "spot tests" to confirm some of the microorganisms, and always as a presumptive diagnosis.

Quesada Muñiz and Rodriguez Martinez get protection (Author's Certificate of invention No. 20000083) for a formulation that includes a mixture of protein hydrolysates, proteins, alcohols and other elements which allows, besides identifying the organisms of interest, such as *E. coli and Salmonella*, the identification of *Pseudomonas aeruginosa* in less than 24 h. However the medium was unable to facilitate the identification of other Gram-negative organisms of interest that grew as colorless colonies.

Roth and collaborators in 1993 protected a method and a chromogenic medium for the identification and differentiation of total coliforms and *E. coli*. (Patent No. US 5 393 662). The medium is composed of substrates with different chromophore groups. This medium did not facilitate the identification and count of *E. coli* O157:H7, *Salmonella* and other non-coliform enterobacteria. *Shigella sonnei* causes positive false results since in the medium the colonies grew with the same color characteristics than *E. coli*.

In 1997, KAI A. Hengstler and coworkers. (KAI A. Hengstler, Rainer Hammann and

Anne Marie Fahr. Evaluation of BBL CHROMagar Orientation Medium for Detection and Presumptive Identification of Urinary Tract Pathogens. Journal of Clinical Microbiology, Nov., 1997, p. 2773-2777) evaluated the CHROMagar Orientation medium, for the detection and presumptive identification of pathogens in urine samples. The medium allowed the detection of several species of microorganisms of interest by the coloration of the colonies, among them: rosy to red (*E. coli*), Blue violet *(Klebsiella spp)*, Blue-red *(Enterobacter spp.)*, Rose *(Enterobacter spp*., *Citrobacter freundii, Proteus mirabilis)*, Blue *(Serratia spp.),* Blue-violet *(Citrobacter freundii and koseri)*, Colorless to beige with *Brown* medium *(Morganella morganii, Proteus mirabilis and vulgaris),* Blue with Brown medium *(Proteus vulgaris),* colorless in Brown medium *(Enterococcus spp.),* Blue brilliant *(Streptococcus agalactiae)*, Green blue *(Enterococcus spp.)* and Light rose *(Staphylococcus saprophyticus*).

As it is appreciated from the prior art, each microorganism cannot be correctly identified, since it does not respond to a single color pattern, for what one can affirm that the identification can not only be carried out by the attributes of the colony or of the medium, and they are required of other tests, such it is the case of *Proteus mirabilis, Enterobacter, Citrobacter freundii, Proteus vulgaris, Enterococcus spp.* In another aspect the medium does not facilitate a correct identification, since different species present the same coloration, as in the case of *Enterobacter spp*., *Citrobacter freundii* and *Proteus Mirasbilis* that appear all with a pink color.

In March of 1998, Roth and collaborators patented an invention (Patent No. US 5,726,031) in which was reported a medium and a quantitative method for the identification and differentiation of biological material in a test sample. This medium includes an specific chromogenic substrate to one of the biological materials that grants a coloration to that material, a second chromogenic substrate specific for a second type of biological material and which facilitates the obtainment of a second coloration different to the first one characteristic for colonies of another species, and a third biological material to be tested that split one of the two substrates. The first one and second biological materials degrade a sugar, and the third biological material does not degrade that sugar.

In the medium a pH indicator is included, and it allows the change of the color of the medium, as a result of the hydrolysis of the sugar around the colony and the colony takes the characteristic colors of the chromogenic substrates splitting. The main components are: the 6-chloro-3-indolyl galactoside, 5-bromo-4-chloro-3-indolyl glucuronide, sorbitol and phenol red. Also, the bile salts, sodium lauryl sulfate, sodium desoxycholate, polyglycol ether and antibiotics derived from acriflavine are included.

The composition also includes an inductor of the enzymatic reactions (isopropyl-beta- D-thiogalactopyranoside), agars, pectins, carragenines, alginates, xantin and peptones. This invention can be considered as the nearest analogue to the present invention.

The inconveniences of the mentioned invention can be resumed as follows:
- *Salmonella typhi* cannot be differentiated from *Salmonella* non *typhi*, and its confirmative identification is difficult and for some strains impossible, because in the medium, it can appear as white colonies, such as it happens with other Gram-negative bacteria, such as *Proteus.*
- In the cases in that several species grow in one Petri dish, is very difficult to identify the yellow zones characteristics of most of the *Salmonella,* since this takes place due to the acidification of the medium and the formation of yellow zones can take place by the overlapping of the reactions in the medium.
- For the identification and count of other Gram-negative non-coliforms, such as
   *Pseudomonas, Aeromonas, Klebsiella,* among other, is not possible, because they require the use of other tests for their identification.
- The same as for the conventional media, from 24 to 48 hours as minimum, are needed to identify the organisms of interest.
- The components that should promote the growth of the organisms of interest in the medium, are not enough to allow the early development (before 24 h) of the identification reactions, and it becomes necessary the use of an inductor for the enzyme β-galactosidase (IPTG).
- The sodium dodecylsulfate, the acryflavine and the antibiotics in the medium, make it more complex in preparation and more costly. Its stability is restricted by the presence of antibiotics that are added as supplements.
- The method is carried out incubating the sample at 40 °C, and not at the temperature recommended for most of the organisms and institutions that regulate the microbiological procedures (44 °C) and it can cause problems because of the procedures are not standardized and it can cause confusions for the laboratory personnel.

### Disclosure of the invention

The objective of the present invention consists on providing a new culture medium for the identification and/or differentiation of Gram-negative microorganisms of interest in the microbiological diagnosis in the clinic and the veterinary, the quality control of foods and the monitoring of the contamination of the environment and a method for its use.

The novelty of the invention consists in to provide to the laboratory a culture medium, able to differentiate a considerable quantity of Gram-negative organisms of interest for the diagnosis in a single container (determination), on the base of differences in at least 10 colors of the colonies of different sizes, and of at least 5 types of different halos, by its colors and sizes.

For the first time, the medium allows to evaluate, simultaneously, *E. coli, E. coli* O157:H7 *Salmonella typhi* and non *typhi*, and even between them; *Klebsiella, Shigella* and between them; *Serratia*, *Enterobacter, Proteus, Pseudomonas, Citrobacter* and *Aeromonas*.

Some microorganisms appear with characteristic colors on this medium, such as, *Salmonella typhi* and *S. schotmuleri* that show an orange color, *Aeromonas hydrophila* with light green color, *Pseudomonas aeruginosa* orange-rosy color, *Shigella sonnei* with reddish violet color, *Shigella flexneri* translucent and with orange to yellow color, *Serratia odorifera* with violet greenish color and *Proteus and Providence as colorless colonies*.

Unexpected findings were the characteristic halos of certain microorganisms, as the case of *Pseudomonas aeruginosa* with a wide transparent halo, *Shigella sonnei* with a yellow halo, *Klebsiella pneumoniae* with a rosy beige halo, *Serratia* with a small transparent halo, *Aeromonas hydrophila* with a transparent halo and *Salmonella typhimurium* with an orange halo.

Some unexpected responses of the microorganisms to detect were obtained, when using the phenol red as indicator, specifically, the colonies of *Shigella sonnei* of blue color, irregular borders and medium of strawberry rosy color, *Pseudomonas aeruginosa-* colonies of greenish beige color and medium of rosy color and

*Salmonella typhimurium-* colonies of beige color or colorless and medium of strawberry rosy color.

On the other hand, new combinations of elements are offered that allow obtaining well-differentiated halos, not described previously before for the mentioned organisms, as the quantities of siliceous earth, skimmed milk, starches and activated charcoal that are added to the medium in the proposed proportions.

The substances that, jointly with the ingredients described in the previous paragraph, and mixed to each other, guarantee the appearance of different colors in the colonies and the halos, selected among the propylene glycol (from 5 to 15 mL/L), neutral red (up to 0,05 g/L), phenol red (up to 0,05 g/L), magenta glucuronide (from 0,05 to 0,25 g/L), X-gal (from 0,03 to 0,1 g/L) and MUG (up to 0,07 g/L), are part of the originality, because in those combinations and proportions guarantee the appearance of colors in the colonies and halos previously not described.

The quantities of the three nutrient bases in the medium in relation to the content of inhibitors of the growth of the Gram-negative organisms, offer a new combination able to promote the growth of competitive organisms.

The advantages of the proposed medium and the method, described in the present invention, are detailed as fallows:
- The medium not only allows the differentiation of the coliforms in general, but specifically among most of them, those of more diagnostic importance in the case of the clinic and of the quality control of foods.
- With this medium a previously not described great variety of different genera and species can be identified simultaneously in a single Petri dish, with the consequent saving of laboratory resources, reduction of the time for preparation of materials and economy of personal.
- The medium is not sterilized and its preparation is simple.
- The method allows during its application, the incubation of the sample in a wide range of temperature, even higher than 40 °C, since the duration of this stage, is only of 18 hours and the medium does not suffer deterioration.
- The high content of solids in the medium, the nature of their components, and their pH, guarantee the preservation of the same one for enough periods of time, enough to maintain a stock in the laboratory, ready for their use.
- Until the moment, false positive or false negative reactions have not been reported for the tested microorganisms, responding all to the identification patterns that have been proposed for the medium, it means that the colors of the colonies, of the centers, of the halos and the sizes and forms of the borders are characteristic and they do not vary.
- There are not any substance in the medium which can cause a fast deterioration, due to their capacity to be oxidized, and on the contrary, it contains substances that help to preserve the viability of the target microorganisms, and they even protect the cells of damages caused by chemical agents, as the case of the charcoal, milk, starch and siliceous earth.
- With the medium and the method described, the differentiation is achieved, not only for *Salmonella* non *typhi* from *typhi*, but for some of them to each other.
- When identifying the organisms mainly in the area of growth, that is to say for the color of the colonies and their center, of their halo and their morphology, the identification procedure depends in a less significant way from the color change reactions in the medium, which can provoke a wrong identification when reactions are overlapped, for example, when testing polluted samples with a wide range of organisms of different species and genera, as in the case of the strongly polluted or putrid waters.
- Additional tests are not necessary to identify organisms of sanitary interest, such as *Pseudomonas*, *Aeromonas,* and *Klebsiella.*
- The nutritive bases employed in the proposed quantities, jointly with the presence of protective agents and having in consideration the concentration of the inhibitors; facilitate a fast growth and recovery, before 24 hours, of all the organisms of interest.

### Detailed description of the invention

The present invention provides a culture medium for the identification of Gram-negative microorganisms which comprises a mixture of compounds that provide the appearance of halos of different colors and sizes, constituted by siliceous earth, skimmed milk, starches and bacteriological charcoal. Said medium also comprises a mixture of nutrient bases, substances that guarantee the appearance of different colors of the colonies, substances that guarantee the inhibition of the Gram-positive organisms and substances that provide a solid matrix for the growth and development of the colonies.

Within the culture medium of the invention, the compounds that provide the appearance of halos of different colors and sizes are in the medium in quantities from 8 to 20 g/L, are in the following amounts:
siliceous earth from 2 to 10 g/L
skimmed milk from 2 to 20 g/L
starch up to 4 g/L
bacteriological charcoal up to 4 g/L

The mixture of nutrient bases contained in the medium is in quantities from 10 to 38 g/L, which is composed by:
Peptones from 2 to 15 g/L
Triptones from 2 to 15 g/L
Yeast extract from 2 to 8 g/L

In the medium of the invention, the substances that guarantee the appearance of different colors of the colonies are chosen from the group consisting in propylene glycol, which is used in amounts from 5 to 15 mL/L; neutral red, which is used in amounts up to 0,05 g/L; phenol red, which is used in amounts up to 0,05 g/L; magenta glucuronide, which is used in amounts from 0,05 to 0,25 g/L; X-gal, which is used in amounts from 0,03 to 0,1 g/L and MUG, which is used in amounts up to 0,07 g/L.

On the other hand the substances that guarantee the inhibition of the Gram-positive organisms are in quantities from 0,1 to 1 g/L, preferably being used sodium desoxycholate.

Additionally the medium posses substances that provide a solid matrix for the growth and development of the colonies which are composed by the combination of the mixture of compounds that provide the appearance of halos of different colors and sizes, particularly siliceous earth, skimmed milk, starches and bacteriological charcoal with agar, in proportions from 0,75:1 to 2:1.

The invention is also related with a method for the identification of Gram-negative microorganisms, comprising the step of contacting the microorganisms with the culture medium described above, wherein the differentiation of the organisms of interest is through the appearance of at least 10 characteristic colors of the regular and irregular colonies, and of halos of at least 5 different characteristic colors and sizes.

In the referred method the identification of the different organisms is made as follows:
- *E. coli* by the appearance of colonies of intense violet bluish color and blue halo and medium of orange color and in certain cases, fluorescence of blue color;
- *E. coli* O157:H7 by the appearance of colonies of violet bluish or greenish color and medium of rosy color;
- *Shigella sonnei* by the appearance of colonies of violet reddish color, very irregular borders and yellow halo;
- *Shigella flexneri* by the appearance of translucent colonies of orange to yellow color, mucoids and medium of orange to yellow color;
- *Pseudomonas aeruginosa* by the appearance of colonies of orange-rosy color, transparent halo and greenish fluorescence before 24 hours and greenish color after 24 hours;
- *Klebsiella pneumoniae* by the appearance of colonies of violet reddish color, mucoids with rosy beige halo in occasions;
- *Serratia odorifera* and *Serratia marcencens* by the appearance of colonies of violet greenish color and transparent very small halo;
- *Proteus mirabilis, Proteus vulgaris and Providence spp* by the appearance of colorless small colonies and medium of orange color;
- *Salmonella enteritidis* by the appearance of colonies of red color and regular borders ;
   *Salmonella cholerasuiss* by the appearance of colonies of red color and irregular borders;
- *Salmonella typhimurium* by the appearance of colonies of red color and halo of variable orange color;
- *Salmonella schotmuelleri* by the appearance of colonies of orange color, translucent and medium of orange to yellow color;
- *Salmonella typhi* by the appearance of colonies of orange color and yellow medium;
- *Enterobacter aerogenes* and *E. cloacae* by the appearance of colonies of light violet or violet greenish and center of more intense violet color;
- *Citrobacter freundii* by the appearance of small colonies of dark violet color and center of more intense violet color;
- *Aeromonas hydrophila* by the appearance of colonies of light green color and wide transparent halo.

When the identification of different organisms using phenol red is carried out, the following results are obtained:
- *E. coli* by is identified by the appearance of colonies of blue color and medium of rosy color and in the case of using MUG, fluorescence of blue color;
- *Shigella sonnei* is identified by the appearance of colonies of blue color, irregular borders and medium of strawberry rosy color;
- *Pseudomonas aeruginosa* is identified by the appearance of colonies of greenish beige color and medium of rosy color;
- *Salmonella typhimurium* is identified by the appearance of colonies of beige color or colorless and medium of strawberry rosy color;

The culture medium is prepared mixing from 30 to 50 grams of the medium with 1 liter of distilled or deionized water, stirring, boiling until complete melting of the agar, cooling to 45-50°C, adding propylene glycol in quantities from 5 to 15 mL, stirring and distributing in dishes constantly shaking. Then, the samples or the microorganisms are inoculated and incubated at temperature from 30 to 45 °C, for up to 18 hours, identifying or differentiating finally the organisms fundamentally by the characteristics of the color of the colonies, of their center, halo, borders and in the case that is required, by the color of the medium.

At industrial scale the medium gets ready starting from the mixture of the dehydrated ingredients, previously milled and sifted. The mixture is carried out in homogenizers for from 0,5 to 6 hours. A sample is taking and the pH is verified. The pH is adjusted from 6,6 to 7,4 with sodium carbonate, is mixed again from 0,5 to 6 hours. Once the pH is again determined, the medium is submitted to physicochemical and functional control, and if the results are satisfactory, it is filled in flasks of different volumes.

In the laboratory, or in form of a ready to use medium, it proceeds as follows:

In an Erlenmeyer flask firstly, a small volume of distilled or deionized water is poured, taken it from the total volume of 1 Liter, necessary for the preparation of the medium. This quantity of water is mixed with 10 mL of propylene glycol. Subsequently, the dehydrated ingredients are weighed and added, beginning with the agents that faster could gain in moisture, such as the nutrient bases, in quantities from 10 to 30 g, specifically, the peptones from 2 to 15 g/L, the tryptones from 2 to 15 g/L and the yeast extracts from 2 to 8 g/L. Next the ingredients that provide the appearance of halos are added in quantity from 8 to 20 g/L, specially the skimmed milk, from 2 to 20 g/L, the starches up to 4 g/L, the activated charcoal up to 4 g/L and finally the siliceous earth from 2 to 10 g/L.

After that most of the ingredients that guarantee the appearance of the colorations are added, such as the neutral red in quantities up to 0,05 g/L or the phenol red in quantities up to 0,05 g/L; the magenta glucuronide in quantities from 0,005 to 0,02 g/L; the X-gal from 0,003 to 0,005 g/L and the MUG up to 0,002 g/L.

Next, the inhibitors of the Gram-positive organisms are added, preferably the sodium deoxycholate in quantity from 0,1 to 1 g/L.

Lastly the agar is added in a proportion from 0,5:1 to 1,5:1 with regard to the sum of the quantities of milk, starches, charcoal and siliceous earth.

All the ingredients finally should be added in quantities between 30 and 50 g.

The mixture is settle for several minutes and later the rest of the water is added until completing 1 liter, shaking the components well and allowing to settle for an interval of up to 15 min. so that the agar swells.

Then the mixture is heated, always shaking the mixture until boiling and until achieving the complete melting of the agar.

The mixture is set to cool down up to 45-50 °C, the propylene glycol is added in quantities from 5 to 15 mL, the medium is kept under constant agitation and it is distributed in Petri dishes, always shaking.

The dishes are inoculated with the test samples, by any of the established methods, preferably by the poured plate method or by streaking.

Next some examples are presented.

### Example No. 1

1000 g of the powdered dehydrated culture medium are prepared with the following composition:

| | g/1000 g of medium |
|---|---|
| Bovine Muscle Peptone | 118,5 |
| Casein Triptone | 118,5 |
| Yeast extract | 94,8 |
| Skim Milk Powder | 237,0 |

These components were previously sifted.

In the composition the sodium deoxycholate was included as an inhibitor (23,7 g).

It was prepared a pre-mixture of 47,4 g of siliceous earth, with 1,2 g of X-gal and 0,7 g of neutral red and 3,0 g of Magenta glucuronide. After that, all the ingredients were mixed with agar as a gelling agent in quantity of 355 g and sodium carbonate in quantity of 1 g. Once achieved the uniformity of the composition and the pH adjusted at 7,0, this was packed in tightly closed flasks with 21 g of the composition.

At the same time, the propylene glycol was packed in flasks by 5 mL.

The content of the flask with the composition was poured in an Erlenmeyer flask which contained a mixture of deionized water and the content of the propylene glycol flask; the mixture was stirred, allowing it to swell for 10 minutes, and then proceeded to boil for 3 minutes; to cool down until the temperature of 45 °C and to distribute in Petri dishes. Once the composition gelled, the medium was tested in order to evaluate its characteristics and performance.

The physicochemical and organoleptic evaluations are shown in Table No. 1.

**Table No.1 Physicochemical and organoleptic evaluation**

| **Assay** | **Result** |
|---|---|
| Color of the powder | Rosy beige |
| Appearance of the powder | Fine, fluid, homogeneous |
| Color of the prepared medium after melting | Red |
| Transparency of the prepared medium after melting | Opalescent |
| Loss on drying | 6,65 |
| pH of the prepared medium before melting | 6,97 |
| pH of the prepared medium after melting | 6,96 |

The differentiation of the colonies and the promotion of the growth in comparison with general purpose and differential medium for the microorganism of interest were evaluated with certified strains, these were:

Red Violet Bile Agar: Prepared at a concentration of 38,5 g/L in water, was mixed and heated until boiling, cooled down up to 45-50°C and distributed in dishes. S.S. Agar: Prepared at a concentration of 60 g/L in deionized water, mixed and heated until boiling, cooled down up to 45-50°C and distributed in dishes.

In the Table No. 2 can be observed the characteristics of the growth of different microorganisms, as well as, the counts at the dilutions 10⁻⁵ and 10⁻⁶

These, results were satisfactory, in both, the medium object of the present invention, and in the reference media, not showing any inhibition of species of *Salmonella* and *Shigella*. The superiority for the growth of *Shigella sonnei,* was demonstrated for the test medium in comparison with the S.S Agar, what was also evidenced by the number and size of the colonies. In relation to the differentiation of the different species it was achieved, in all the cases, characteristic answers for each species.

A similar assay was carried out with species of the coliform group, using as reference medium the Red Violet Bile Agar medium, as shown in Table No. 3.

**Table No. 2 Differentiation and promotion of the growth of the culture medium developed according to the invention (Salmonella and Shigella)**

| Microorganism | Medium | Average of the counts (CFU/mL) | | Color of the medium | Color of the colonies | Morphology of the colonies |
|---|---|---|---|---|---|---|
| | | 10⁻⁵ | 10⁻⁶ | | | |
| *Salmonella typhimurium* ATCC 14028 | Experimental | NC | 325 | Rosy | Red | Regular borders, ≈2 mm |
| | S.S. AGAR | NC | 210 | Orange | Colorless | Regular borders, I center black, ≈2 mm |
| *Salmonella enteritidis* ATCC 13076 | Experimental | 195 | 20 | Rosy | Red | Regular borders, ≈ 2 mm |
| | S.S. AGAR | 235 | 20 | Orange | Colorless | Regular borders, ≈1-2 mm |
| *Shigella flexneri* ATCC 12022 | Experimental | NC | 75 | Yellowish orange | Yellow, translucent | Regular borders, ≈ 2 mm |
| | AGAR S.S. | NC | 30 | Orange | Colorless | Regular borders, ≈2 mm |
| *Shigella sonnei* ATCC 25931 | Experimental | 155 | 20 | Rosy Orange | Reddish Violet | Borders very irregular, ≈ 5 mm |
| | S.S. AGAR | NG | NG | NG | NG | NG |

| | | | | | | |
|---|---|---|---|---|---|---|
| NC: Countless | | | | | | |
| NG: Non growth | | | | | | |
| CFU/mL: Colony Forming Units for each mL of the dilution | | | | | | |

**Table No. 3 Differentiation and promotion of the growth (Coliforms)**

| Microorganism | Medium | Average of the counts (CFU/mL) | | Color of the medium | Color of the colonies | Morphology of the colonies |
|---|---|---|---|---|---|---|
| | | 10⁻⁵ | 10⁻⁶ | | | |
| *Escherichia coli* ATCC 25922 | Experimental | NC | 235 | Rosy | Intense bluish violet, with blue halo | Regular borders, ≈2 mm |
| | Violet Red Bile Agar | INC | 155 | Rosy violet | Red violet, with bile precipitate | Regular borders, ≈3 mm |
| *Enterobacter aerogenes* ATCC 13048 | Experimental | NC | 245 | Rosy | Light violet with dark center | Regular borders, ≈ 2-3 mm |
| | Violet Red Bile Agar | NC | 205 | Rosy violet | Violets | Regular borders, ≈ 2mm |

| | | | | | | |
|---|---|---|---|---|---|---|
| NC: Countless | | | | | | |
| NG: Non growth | | | | | | |
| CFU/mL: Colony Forming Units for each mL of dilution | | | | | | |

The counts of these two microorganisms in the medium object of the invention, and in the medium used as reference, were the same. The differentiation of the microorganisms was achieved by its colors and morphological characteristics in both cases.

There were inoculated a group of strains by streaking until obtaining isolated colonies in the surface of the medium, what allowed the differentiation of other 5 species according to the table No.4.

**Table No. 4 Differentiation of other Gram-negative species in the medium**

| Microorganism | Color of the medium | Color of the colonies | Morphology of the isolated colonies |
|---|---|---|---|
| *Salmonella schotmuleri* ATCC 10719 | Orange | Orange | Regular borders, (2-3 mm) |
| *Klebsiella pneumoniae* ATCC 13883 | Rosy | Violet reddish | Mucoids, (2 mm) |
| *Citrobacterfreundii* ATCC 8090 | Rosy | Intense violet | Regular borders, (1 mm) |
| *Enterobacter cloacae* ATCC 23355 | Rosy | Light violet with dark center | Regular borders, ≈ 2-3 mm |
| *Escherichia coli* O157:H7 ATCC 35150 | Rosy | Intense bluish violet without halo around | Regular borders, (2 mm) |

A very good differentiation ot all the microorganisms was observed in me medium, standing out the difference among the strain *of E. coli* O157:H7 *and the* typical *E. coli*, other coliforms like *Klebsiella* and *Enterobacter*, showed characteristic colorations.

### Example No. 2

The formulation was prepared weighing the ingredients separated each from another in an Erlenmeyer flask in quantities to prepare 100 mL of medium, the ingredients were used in concentrations according to the example No.1 except for the siliceous earth, of which a concentration of 2 g/L was used. 100 mL of deionized water previously blended with 1 mL of propylene glycol was added. The pH value was adjusted at 6,94 and the later preparation was carried out as it is described in the example No.1.

The tested strains of microorganisms that produce a translucent halo in the surroundings of the colonies were inoculated by streaking until obtaining isolated colonies. They were incubated 24 h at 37 °C. The results are observed in the table No 5.

**Table No. 5 Results of the formulation with 2 g/L of siliceous earth**

| Microorganism | Color of the medium | Color of the colonies | Morphology of the isolated colonies |
|---|---|---|---|
| *Pseudomona aeruginosa* ATCC 27853 | Orange | Rosy, fluorescent with translucent halo of ≈ 3 mm | Mucoids, lightly irregular ≈ 2-3 mm |
| *Aeromona hydrophila sp.* | Orange | Light green with translucent halo of ≈ 3 mm | Regular borders, mucoids, ≈ 2 mm |
| *Serratia marcencens* ATCC 8100 | Yellow orange | Violet greenish with small halo of ≈ 1 mm | Regular borders, ≈1-2 mm |

A good differentiation of the microorganisms was observed by color and morphology, being very visible the translucent halo around the colonies.

### Example No. 3

The medium was formulated weighing the ingredients separately in an Erlenmeyer flask, according to the concentrations described in the example 1, except for the substrate Magenta Glucuronide, of which a concentration of 1,5 µg/mL was used, the later preparation was carried out according to the example No.2 and it was observed a response of a group of enterobacteria in the formulation as it is shown in the Table No. 6.

**Table No. 6 Characteristics of the growth of enterobacteria in the medium.**

| Microorganism | Color of the medium | Color of the colonies | Morphology of the isolated colonies |
|---|---|---|---|
| *Shigella flexneri* ATCC 12022 | Yellow | Yellow, translucent | Mucoids, lightly irregular borders, ≈ 2-3 mm |
| *Shigella sonnei* ATCC 25931 | Orange | Violet reddish | Mucoids, irregular borders, ≈4-5 mm |
| *Proteus mirabilis ATCC 12453* | Orange | Orange with dark center | Lightly irregular borders, ≈2 mm |
| *Providence sp.* | Orange | Orange | Irregular borders, ≈1-3 mm |
| *Proteus vulgaris* ATCC 13315 | Orange | Orange | Irregular borders, ≈ 3-5 mm |
| *Escherichia coli* ATCC 25922 | Orange reddish | Violets with blue halo | Regular borders, center of ≈ 2 mm, halo ≈ 1 mm |
| *Escherichia coli* O157:H7 ATCC 35150 | Orange reddish | Violet greenish | Regular borders, ≈1-2 mm |
| *Salmonella typhimurium* ATCC 14028 | Orange | Red with dark center | Lightly irregular borders, ≈1-2 mm |
| *Salmonella typhi* ATCC 2280 | Orange | Orange | Lightly irregular borders, ≈1-2 mm |
| *Salmonella cholerae-suis* ATCC 10708 | Reddish orange | Red | Irregular borders, mucoids, ≈2-3 mm |
| *Salmonella schotmuleri* ATCC 10719 | Yellow | Light orange | Irregular borders ≈3 mm |
| *Enterobacter cloacae* ATCC 8100 | Orange | Green with violet center | Regular borders, ≈2-3 mm |
| *Enterobacter aerogenes* ATCC 13048 | Orange | Green with violet center | Regular borders, ≈3 mm |

In this example can be observed the functionality of the formulation with a wide group of certified strains of the *Enterobacteriaceae* family. The typical reactions of the medium for the inoculated species of interest were observed with easiness.

### Example No. 4

The medium was formulated weighing the ingredients separately in an Erlenmeyer flask, according to the concentrations described in the example 1, except for the substitution of the substrate Magenta Glucuronide for the fluorogenic substrate MUG, of which a concentration of 0,05 g/L was used, the further preparation was carried out according to the example No.2.

A group of microorganisms was inoculated by streaking to obtain isolated colonies as observed in the Table No.7.

**Table No. 7 Evaluation of the formulation with MUG**

| Microorganism | Color of the medium | Color of the colonies | Morphology of the isolated colonies |
|---|---|---|---|
| *Escherichia coli* ATCC 25922 | Reddish orange | Violets with blue, fluorescent halo | Regular borders |
| *Escherichia coli* 0157:H7 ATCC 35150 | Reddish orange | Dark violet, without halo | Regular borders |
| *Salmonella typhimurium* ATCC 14028 | Red | Red | Lightly irregular borders |

### Example No. 5

The medium was formulated with the following composition:

| Ingredient | g/L |
|---|---|
| Peptone | 5 |
| Triptone | 5 |
| Yeast extract | 4 |
| X-gal | 0,05 |
| Magenta-glucuronide | 0,1 |
| Desoxycholate | 1 |
| Insoluble starch | 4 |
| Phenol Red | 0,018 |
| Agar | 15 |

The ingredients were weighed in an Erlenmeyer flask, 1 L of deionized water was added, blended with 10 mL of propylene glycol, the pH was adjusted at 7.0, heated under continuous stirring until boiling and it was placed for 15 minutes in an autoclave without pressure. The medium was allowed to cool down up to 45-50 °C and it was distributed in Petri dishes

Enterobacteria strains were inoculated by streaking until obtaining isolated colonies. The results are showed in the Table No. 8.

**Table No. 8 Evaluation of the formulation with starch and phenol red**

| Microorganism | Color of the medium | Color of the colonies | Morphology of the isolated colonies |
|---|---|---|---|
| *Escherichia coli* ATCC 25922 | Rosy | Blue | Regular borders |
| *Pseudomonas aeruginosa* ATCC 27853 | Rosy | Beige greenish | Lightly irregular borders |
| *Salmonella typhimurium* ATCC 14028 | Rosy | Beige | Regular borders |
| *Shigella sonnei* ATCC 25931 | Rosy | Blue | Irregular borders |

## Claims

1. Culture medium for the identification of Gram-negative microorganisms which comprises a mixture of compounds that provide the appearance of halos of different colors and sizes, constituted by siliceous earth, skimmed milk, starches and bacteriological charcoal, and also comprising a mixture of nutrient bases, substances that guarantee the appearance of different colors of the colonies, substances that guarantee the inhibition of the Gram-positive organisms and substances that provide a solid matrix for the growth and development of the colonies.

2. Culture medium according to claim 1 wherein the compounds that provide the appearance of halos of different colors and sizes are in the medium in quantities from 8 to 20 g/L, and particularly each component is in the following amounts:
siliceous earth from 2 to 10 g/L
skimmed milk from 2 to 20 g/L
starch up to 4 g/L
bacteriological charcoal up to 4 g/L

3. Culture medium according to claim 1 wherein the mixture of nutrient bases is in quantities from 10 to 38 g/L and it is composed by:
Peptones from 2 to 15 g/L
Triptones from 2 to 15 g/L
Yeast extract from 2 to 8 g/L

4. Culture medium according to claim 1 wherein the substances that guarantee the appearance of different colors of the colonies are chosen from the group consisting in propylene glycol, which is used in amounts from 5 to 15 mL/L; neutral red, which is used in amounts up to 0,05 g/L; phenol red, which is used in amounts up to 0,05 g/L; magenta glucuronide, which is used in amounts from 0,05 to 0,25 g/L; X-gal, which is used in amounts from 0,03 to 0,1 g/L and MUG, which is used in amounts up to 0,07 g/L.

5. Culture medium according to claim 1 wherein the substances that guarantee the inhibition of the Gram-positive organisms are in quantities from 0,1 to 1 g/L, preferably being used sodium desoxycholate.

6. Culture medium according to claim 1 wherein the substances that provide a solid matrix for the growth and development of the colonies is the combination of the mixture of compounds that provide the appearance of halos of different colors and sizes, particularly siliceous earth, skimmed milk, starches and bacteriological charcoal with agar, in proportions from 0,75:1 to 2:1.

7. Method for the identification of Gram-negative microorganisms, comprising the step of contacting the microorganisms with the culture medium of claim 1, wherein the differentiation of the organisms of interest is through the appearance of at least 10 characteristic colors of the regular and irregular colonies, and of halos of at least 5 different characteristic colors and sizes.

8. Method according to claim 7, wherein the identification of the different organisms is made as follows:
- *E. coli* by the appearance of colonies of intense violet bluish color and blue halo and medium of orange color and in certain cases, fluorescence of blue color;
- *E. coli* O157:H7 by the appearance of colonies of violet bluish or greenish color and medium of rosy color;
- *Shigella sonnei* by the appearance of colonies of violet reddish color, very irregular borders and yellow halo;
- *Shigella flexneri* by the appearance of translucent colonies of orange to yellow color, mucoids and medium of orange to yellow color;
- *Pseudomonas aeruginosa* by the appearance of colonies of orange-rosy color, transparent halo and greenish fluorescence before 24 hours and greenish color after 24 hours;
- *Klebsiella pneumoniae* by the appearance of colonies of violet reddish color, mucoids with rosy beige halo in occasions;
- *Serratia odorifera* and *Serratia marcencens* by the appearance of colonies of violet greenish color and transparent very small halo;
- *Proteus mirabilis, Proteus vulgaris and Providence spp* by the appearance of colorless small colonies and medium of orange color;
- *Salmonella enteritidis* by the appearance of colonies of red color and regular borders ;
*Salmonella cholerasuiss* by the appearance of colonies of red color and irregular borders;
- *Salmonella typhimurium* by the appearance of colonies of red color and halo of variable orange color;
- *Salmonella schotmuelleri* by the appearance of colonies of orange color, translucent and medium of orange to yellow color;
- *Salmonella typhi* by the appearance of colonies of orange color and yellow medium;
- *Enterobacter aerogenes* and *E. cloacae* by the appearance of colonies of light violet or violet greenish and center of more intense violet color;
- *Citrobacter freundii* by the appearance of small colonies of dark violet color and center of more intense violet color;
- *Aeromonas hydrophila* by the appearance of colonies of light green color and wide transparent halo.

9. Method according to claim 7, wherein the identification of different organisms when using phenol red is made as follows:
- *E. coli* by the appearance of colonies of blue color and medium of rosy color and in the case of using MUG, fluorescence of blue color;
- *Shigella sonnei* by the appearance of colonies of blue color, irregular borders and medium of strawberry rosy color;
- *Pseudomonas aeruginosa* by the appearance of colonies of greenish beige color and medium of rosy color;
- *Salmonella typhimurium* by the appearance of colonies of beige color or colorless and medium of strawberry rosy color;

10. Method according to claims 7 to 9, wherein the culture medium is prepared by mixing from 30 to 50 grams of the medium with 1 liter of distilled or deionized water, stirring, boiling until complete melting of the agar, cooling to 45-50 °C, adding propylene glycol in quantities from 5 to 15 mL, stirring and distributing in dishes constantly shaking, then the samples of the microorganisms are inoculated and incubated at temperature from 30 to 45 °C, for up to 18 hours, and finally they are identified or differentiated by the characteristics of the color of the colonies, of their center, halo, borders and in the case that is required, by the color of the medium.

## Patentansprüche

1. Kulturmedium zur Identifikation von Gram-negativen Mikroorganismen, das eine Mischung von Verbindungen enthält, die das Auftreten von Halos mit unterschiedlichen Farben und Größen bereitstellt, gebildet aus Kieselgur, Magermilch, Stärken und bakteriologischer (Aktiv)Kohle, und das auch eine Mischung aus Nährstoffbasen, Substanzen, die das Auftreten von unterschiedlichen Farben der Kolonien garantieren, Substanzen, die die Inhibierung der Gram-positiven Organismen garantieren, und Substanzen enthält, die eine feste Matrix für das Wachstum und die Entwicklung der Kolonien bereitstellen.

2. Kulturmedium nach Anspruch 1, worin die Verbindungen, die das Auftreten von Halos mit unterschiedlichen Farben und Größen bereitstellen, in dem Medium in Mengen von 8 bis 20 g/L vorhanden sind, und worin insbesondere jede Komponente in den folgenden Mengen vorhanden ist:
Kieselgur von 2 bis 10 g/L
Magermilch von 2 bis 20 g/L
Stärke bis zu 4 g/L
bakteriologische (Aktiv)Kohle bis zu 4 g/L

3. Kulturmedium nach Anspruch 1, worin die Mischung an Nährstoffbasen in Mengen von 10 bis 38 g/L vorhanden ist und zusammengesetzt ist aus:
Peptone von 2 bis 15 g/L
Triptone von 2 bis 15 g/L
Hefeextrakt von 2 bis 8 g/L

4. Kulturmedium nach Anspruch 1, worin die Substanzen, die das Auftreten von unterschiedlichen Farben der Kolonien garantieren, ausgewählt sind aus der Gruppe, bestehend aus Propylenglykol, das in Mengen von 5 bis 15 mL/L verwendet wird; Neutralrot, das in Mengen bis zu 0,05 g/L verwendet wird; Phenolrot, das in Mengen bis zu 0,05 g/L verwendet wird; Magenta-Glucuronid, das in Mengen von 0,05 bis 0,25 g/L verwendet wird; X-gal, das in Mengen von 0,03 bis 0,1 g/L verwendet wird; und MUG, das in Mengen bis zu 0,07 g/L verwendet wird.

5. Kulturmedium nach Anspruch 1, worin die Substanzen, die die Inhibierung der Gram-positiven Organismen garantieren, in Mengen von 0,1 bis 1 g/L vorhanden sind, wobei bevorzugt Natriumdesoxycholat verwendet wird.

6. Kulturmedium nach Anspruch 1, worin die Substanzen, die eine feste Matrix für das Wachstum und die Entwicklung der Kolonien bereitstellen, die Kombination der Mischung der Verbindungen ist, die das Auftreten von Halos unterschiedlicher Farben und Größen bereitstellen, insbesondere Kieselgur, Magermilch, Stärken und bakteriologische (Aktiv)Kohle, mit Agar, in Verhältnissen von 0,75:1 bis 2:1.

7. Verfahren zur Identifikation von Gram-negativen Mikroorganismen, das den Schritt des in Kontakt Bringens der Mikroorganismen mit dem Kulturmedium nach Anspruch 1 enthält, worin die Differentiation der interessierenden Organismen durch das Auftreten von wenigstens 10 charakteristischen Farben der regelmäßigen und unregelmäßigen Kolonien erfolgt, und durch Halos mit wenigstens 5 unterschiedlichen charakteristischen Farben und Größen.

8. Verfahren nach Anspruch 7, worin die Identifikation der unterschiedlichen Organismen wie folgt erfolgt:
- *E. coli* durch das Auftreten von Kolonien mit intensiver violett-bläulicher Farbe und blauem Halo und einem Medium mit oranger Farbe und, in bestimmten Fällen, Fluoreszenz mit blauer Farbe;
- *E. coli* O157:H7 durch das Auftreten von Kolonien mit violett-bläulicher oder grünlicher Farbe und einem Medium mit rosa Farbe;
- *Shigella sonnei* durch das Auftreten von Kolonien mit violett-rötlicher Farbe, sehr unregelmäßigen Rändern und gelbem Halo;
- *Shigella flexneri* durch das Auftreten von transluzenten Kolonien mit oranger bis gelber Farbe, Mucoiden und Medium mit oranger bis gelber Farbe;
- *Pseudomonas aeruginosa* durch das Auftreten von Kolonien mit orangerosa Farbe, transparentem Halo und grünlicher Fluoreszenz vor 24 Stunden und grünlicher Farbe nach 24 Stunden;
- *Klebsiella pneumoniae* durch das Auftreten von Kolonien mit violett-rötlicher Farbe, gelegentlich Mucoiden mit rosa-beigem Halo;
- *Serratia odorifera* und *Serratia marcencens* durch das Auftreten von Kolonien mit violett-grünlicher Farbe und sehr kleinem transparentem Halo;
- *Proteus mirabilis, Proteus vulgaris* und *Providence spp* durch das Auftreten von kleinen farblosen Kolonien und Medium mit oranger Farbe;
- *Salmonella enteritidis* durch das Auftreten von Kolonien mit roter Farbe und gleichmäßigen Rändern;
- *Salmonella cholerasuiss* durch das Auftreten von Kolonien mit roter Farbe und unregelmäßigen Rändern;
- *Salmonella typhimurium* durch das Auftreten von Kolonien mit roter Farbe und Halo mit variabler oranger Farbe;
- *Salmonella schotmuelleri* durch das Auftreten von Kolonien mit oranger Farbe, transluzent, und Medium mit oranger bis gelber Farbe;
- *Salmonella typhi* durch das Auftreten von Kolonien mit oranger Farbe und gelbem Medium;
- *Enterobacter aerogenes* und *E. cloacae* durch das Auftreten von Kolonien mit hell-violetter oder violett-grünlicher Farbe und einem Zentrum mit intensiverer violetter Farbe;
- *Citrobacter freundii* durch das Auftreten von kleinen Kolonien mit dunkelvioletter Farbe und einem Zentrum mit intensiverer violetter Farbe;
- *Aeromonas hydrophila* durch das Auftreten von Kolonien mit hell-grüner Farbe und einem breiten transparenten Halo.

9. Verfahren nach Anspruch 7, worin die Identifikation von unterschiedlichen Organismen wie folgt erfolgt, wenn Phenolrot verwendet wird:
- *E. coli* durch das Auftreten von Kolonien mit blauer Farbe und Medium mit rosa Farbe, und im Fall einer Verwendung von MUG, Fluoreszenz mit blauer Farbe;
- *Shigella sonnei* durch das Auftreten von Kolonien mit blauer Farbe, unregelmäßigen Rändern und Medium mit Erdbeer-rosa Farbe;
- *Pseudomonas aeruginosa* durch das Auftreten von Kolonien mit grünlichbeiger Farbe und Medium mit rosa Farbe;
- *Salmonella typhimurium* durch das Auftreten von Kolonien mit beiger Farbe oder farblosen Kolonien und Medium mit Erdbeer-rosa Farbe.

10. Verfahren nach Ansprüchen 7 bis 9, worin das Kulturmedium hergestellt wird durch Mischen von 30 bis 50 Gramm des Mediums mit 1 Liter destilliertem oder deionisiertem Wasser, Rühren, Kochen bis zum vollständigen Schmelzen des Agars, Abkühlen auf 45-50 °C, Zugeben von Propylenglykol in Mengen von 5 bis 15 mL, Rühren und Verteilen in gleichmäßig geschüttelte Schalen, dann werden die Proben der Mikroorganismen angeimpft und bei einer Temperatur von 30 bis 45 °C für bis zu 18 Stunden inkubiert, und schließlich werden sie identifiziert oder differenziert durch die Charakteristika der Farbe der Kolonien, ihres Zentrums, Halo, Ränder, und, sofern es erforderlich ist, durch die Farbe des Mediums.

## Revendications

1. Milieu de culture pour l'identification de micro-organismes Gram-négatifs qui comprend un mélange de composés qui génèrent l'apparition de halos de différentes couleurs et tailles, constitué par de la diatomite, du lait écrémé, des amidons et du charbon bactériologique, et comprenant également un mélange de bases nutritives, de substances qui garantissent l'apparition de différentes couleurs de colonies, de substances qui garantissent l'inhibition des organismes Gram-positifs et de substances qui fournissent une matrice solide pour la croissance et le développement des colonies.

2. Milieu de culture selon la revendication 1, dans lequel les composés qui génèrent l'apparition de halos de différentes couleurs et tailles sont présents dans le milieu dans des quantités comprises entre 8 et 20 g/L, et en particulier chaque composant est présent dans les quantités suivantes :
entre 2 et 10 g/L de diatomite
entre 2 et 20 g/L de lait écrémé
jusqu'à 4 g/L d'amidon
jusqu'à 4 g/L de charbon bactériologique

3. Milieu de culture selon la revendication 1, dans lequel le mélange de bases nutritives est présent dans des quantités comprises entre 10 et 38 g/L et est constitué de :
2 à 15 g/L de peptones
2 à 15 g/L de triptones
2 à 8 g/L d'extrait de levure

4. Milieu de culture selon la revendication 1, dans lequel les substances qui garantissent l'apparition de différentes couleurs des colonies sont choisies dans le groupe constitué du propylène glycol, qui est utilisé dans des quantités comprises entre 5 et 15 mL/L ; du rouge neutre, qui est utilisé dans des quantités pouvant aller jusqu'à 0,05 g/L ; du rouge de phénol, qui est utilisé dans des quantités pouvant aller jusqu'à 0,05 g/L ; du glucuronide magenta, qui est utilisé dans des quantités comprises entre 0,05 et 0,25 g/L ; du X-gal, qui est utilisé dans des quantités comprises entre 0,03 et 0,1 g/L et de la MUG, qui est utilisée dans des quantités pouvant aller jusqu'à 0,07 g/L.

5. Milieu de culture selon la revendication 1, dans lequel les substances qui garantissent l'inhibition des organismes Gram-positifs sont présentes dans des quantités comprises entre 0,1 et 1 g/L, le désoxycholate de sodium étant de préférence utilisé.

6. Milieu de culture selon la revendication 1, dans lequel les substances qui fournissent une matrice solide pour la croissance et le développement des colonies est la combinaison du mélange de composés qui génèrent l'apparition de halos de différentes couleurs et tailles, en particulier la diatomite, le lait écrémé, les amidons et le charbon bactériologique avec l'agar, dans des proportions allant de 0,75/1 à 2/1.

7. Procédé d'identification de micro-organismes Gram-négatifs, comprenant l'étape consistant à mettre en contact les micro-organismes avec le milieu de culture de la revendication 1, dans lequel la différenciation des organismes d'intérêt est réalisée par l'apparition d'au moins 10 couleurs caractéristiques des colonies régulières et irrégulières, et de halos d'au moins 5 couleurs et tailles caractéristiques différentes.

8. Procédé selon la revendication 7, dans lequel l'identification des différents organismes est effectuée de la manière suivante :
- *E. coli* par l'apparition de colonies de couleur bleuâtre violette intense et d'un halo bleu et d'un milieu de couleur orange et dans certains cas, d'une fluorescence de couleur bleue ;
- *E. coli* O157:H par l'apparition de colonies de couleur bleuâtre ou verdâtre violette et d'un milieu de couleur rose ;
- *Shigella sonnei* par l'apparition de colonies de couleur rougeâtre violette, de contours très irréguliers et d'un halo jaune ;
- *Shigella flexneri* par l'apparition de colonies translucides de couleur orange à jaune, de mucines et d'un milieu de couleur orange à j aune ;
- *Pseudomonas aeruginosa* par l'apparition de colonies de couleur orange à rose, d'un halo transparent et d'une fluorescence verdâtre avant 24 heures et d'une couleur verdâtre après 24 heures ;
- *Klebsiella pneumonia* par l'apparition de colonies de couleur rougeâtre violette, de mucines ayant parfois un halo beige rosé ;
- *Serratia odorifera* et *Serratia marcencens* par l'apparition de colonies de couleur verdâtre violette et d'un très petit halo transparent ;
- *Proteus mirabilis, Proteus vulgaris* et *Providence spp* par l'apparition de petites colonies incolores et d'un milieu de couleur orange ;
- *Salmonella enteritidis* par l'apparition de colonies de couleur rouge et de contours réguliers ;
- *Salmonella cholerasuiss* par l'apparition de colonies de couleur rouge et de contours irréguliers ;
- *Salmonella typhimurium* par l'apparition de colonies de couleur rouge et d'un halo de couleur orange variable ;
- *Salmonella schotmuelleri* par l'apparition de colonies translucides de couleur orange et d'un milieu de couleur orange à jaune ;
- *Salmonella typhi* par l'apparition de colonies de couleur orange et d'un milieu jaune ;
- *Enterobacter aerogenes* et *E. cloacae* par l'apparition de colonies de couleur violet clair ou verdâtre violette et d'un centre de couleur violette plus intense ;
- *Citrobacter freundü* par l'apparition de petites colonies de couleur violet foncé et d'un centre de couleur violette plus intense ;
- *Aeromonas hydrophila* par l'apparition de colonies de couleur vert clair et d'un large halo transparent.

9. Procédé selon la revendication 7, dans lequel l'identification de différents organismes en cas d'utilisation de rouge de phénol est effectuée de la manière suivante :
- *E. coli* par l'apparition de colonies de couleur bleue et d'un milieu de couleur rose et en cas d'utilisation de MUG, d'une fluorescence de couleur bleue ;
- *Shigella sonnei* par l'apparition de colonies de couleur bleue, de contours irréguliers et d'un milieu de couleur rose fraise ;
- *Pseudomonas aeruginosa* par l'apparition de colonies de couleur beige verdâtre et d'un milieu de couleur rose ;
- *Salmonella typhimurium* par l'apparition de colonies de couleur beige ou incolores et d'un milieu de couleur rose fraise.

10. Procédé selon les revendications 7 à 9, dans lequel le milieu de culture est préparé en mélangeant 30 à 50 grammes du milieu avec 1 litre d'eau distillée et déminéralisée, en agitant, en faisant bouillir jusqu'à fusion complète de l'agar, en refroidissant entre 45 et 50 °C, en ajoutant du propylène glycol dans des quantités comprises entre 5 et 15 mL, en agitant et en répartissant dans des récipients à agitation constante, puis les échantillons sont inoculés et incubés à une température comprise entre 30 et 45 °C, pendant un maximum de 18 heures, et enfin ils sont identifiés ou différenciés à l'aide des caractéristiques de couleur des colonies, de leur centre, de leur halo, de leurs contours et dans le cas requis, à l'aide de la couleur du milieu.
